Europäisches Patentamt

European Patent Office

Office européen des brevets

(11) Publication number: **0 216 481 B1**

## EUROPEAN PATENT SPECIFICATION

(45) Date of publication of patent specification: **23.09.92**

(51) Int. Cl.5: **C12P 21/02**, C12N 1/20, C07K 15/04, A01N 63/02, //(C12N1/20,C12R1:07), (C12P21/02,C12R1:07)

(21) Application number: **86306152.9**

(22) Date of filing: **08.08.86**

(54) Polypeptides toxic to larvae of the dipteran family.

(30) Priority: **14.08.85 US 765910**

(43) Date of publication of application:
**01.04.87 Bulletin 87/14**

(45) Publication of the grant of the patent:
**23.09.92 Bulletin 92/39**

(84) Designated Contracting States:
**BE CH DE FR GB IT LI NL SE**

(56) References cited:

**CHEMICAL ABSTRACTS, vol. 96, no. 11, 15th March 1982, page 307, abstract no. 82363q, Columbus, Ohio, US; C.N. CHILCOTT et al.: "The biological significance of proteases in Bacillus thuringiensis var. israelensis crystals", & PROC. UNIV. OTAGO MED. SCH. 1981, 59(2), 40-1 (Cat. D)**

(73) Proprietor: **REPLIGEN CORPORATION**
**101 Binney Street**
**Cambridge Massachusetts 02142(US)**

(72) Inventor: **Witt, Daniel P.**
**288 Essex Street**
**South Hamilton Massachusetts 01982(US)**

(74) Representative: **Smolders, Walter**
**c/o Sandoz AG Patentabteilung**
**CH-4002 Basel(CH)**

Rank Xerox (UK) Business Services

CHEMICAL ABSTRACTS, vol. 100, no. 11, 12th March 1984, page 279, abstract no. 82493h, Columbus, Ohio, US; T. YAMAMOTO et al.: "Mosquitocidal protein of Bacillus thuringiensis subsp. israelensis: identification and partial isolation of the protein", & CURR. MICROBIOL. 1983, 9(5), 279-84 (Cat. D)

CHEMICAL ABSTRACTS, vol. 102, no. 13, 1st April 1985, page 369, abstract no. 109475j, Columbus, Ohio, US; J.M. HURLEY et al.: "Separation of the cytolytic and mosquitocidal proteins of Bacillus thuringiensis subsp. israelensis", & BIOCHEM. BIOPHYS. RES. COMMUN. 1985, 126(2), 961-5

CHEMICAL ABSTRACTS, vol. 103, no. 15, 14th October 1985, page 232, abstract no. 117818j, Columbus, Ohio, US; D.J. ELLAR et al.: "Biochemistry, genetics, and mode of action of Bacillus thuringiensis delta-endotoxins", & MOL. BIOL. MICROB. DIFFER., PROC. INT. SPORE CONF., 9TH 1984 (PUB 1985), 230-40

CHEMICAL ABSTRACTS, vol. 103, no. 21, 25th November 1985, page 278, abstract no. 173977x, Columbus, Ohio, US; P.Y.K. CHEUNG et al.: "Separation of three biologically distinct activities from the parasporal crystal of Bacillus thuringiensis var. israelensis", & CURR. MICROBIOL. 1985, 12(3), 121-6

CHEMICAL ABSTRACTS, vol. 103, no. 23, 9th December 1985, page 234, abstract no. 191329s, Columbus, Ohio, US; P.Y.K. CHEUNG et al.: "Micro-lipid-droplet encapsulation of Bacillus thuringiensis subsp. israelensis delta-endotoxin for control of mosquito larvae", & APPL. ENVIRON. MICROBIOL. 1985, 50(4), 984-8

## Description

Biting flies of the Dipteran family, such as mosquitos, pose a major problem worldwide of both medical and economic importance. Current methods of control include larvaciding, in which control agents are directed against the largely aquatic larvae. These agents often cause significant adverse effects on the environment and, thus, must be used with prudence. Among the pesticides currently in use is the microbially-produced biological insecticide, Bacillus thuringiensis var. israelensis. Upon sporulation, this bacterium produces a proteinaceous crystal containing a toxin which is highly specific to Dipteran larvae. No toxicity to mammals, fish, birds, or even other insects, has been noted. There is concern, however, that viable spores present in the commercial preparations may pose a hazard to the environment. The toxic principle, believed to be a protein, has been the subject of considerable investigation.

Bacillus thuringiensis var. israelensis (Bti) is a spore-forming bacterium first identified in the Negev region of Israel in 1977 as a specific pathogen for mosquito larvae (Goldberg, L.J. and Margalitt, J. [1977] Mosquito News 37:355-358). Its unique properties have led to rapid commercialization as a highly specific control agent for mosquitos and blackflies. A number of attempts to identify and isolate the toxic factor have now been reported. It is known that parasporal crystals from the sporulated bacillus contain the toxic activity and that the spores themselves are not required (Tyrell, D.J., Davidson, L.I., Bulla, L.A., and Ramoska, W.A. [1979] Applied and Environmental Microbiology 38: 656-658). The $LC_{50}$ for mosquito larvae has been reported to be as low as 0.2 ng/ml (based on crystal dry weight). Extraction of these crystals with an alkaline buffer, e.g., 50 mM sodium carbonate, pH 10.5, results in the release from the crystal matrix of the toxic factor(s) into a soluble form (Chilcott, C.N., Kalmakoff, J., and Pillai, J.S. [1981] Proc. Univ. Otago Med. Sch. 59: 40-41; Tyrell, D.J., Bulla, L.A., Andrews, R.E., Kramer, K.J., Davidson, L.I., and Nordin, P. [1981] J. Bact. 145: 1052-1062.) Release is concomitant with a decrease in specific toxicity (greater than ten fold) presumably because the insoluble particulate toxin is more readily assimilated by the filter feeding larvae (Dadd, R.H. [1975] J. Exp. Zool. 191:395-397). Most toxicity is removed although some remains associated with the extracted crystals (Schnell, D., Pfannenstiel, M.A., and Nickerson, K.W. (1984) Science 223:1191-1193). Activity of both the unextracted crystals and the soluble extracts is destroyed by either heat or extensive protease treatment, suggesting that the toxic factor is a protein. Analysis of the proteins present in the soluble extract reveals a major species of MW26000-28000 (26K) in addition to other proteins (Tyrell, D.J., Bulla, L.A., Andrews, R.E., Kramer, K.J., Davidson, L.I., and Nordin, P. [1981] J. Bact. 145:1052-1062; Thomas, W.E. and Ellar, D.J. [1983] J. Cell Sci. 60:181-197). This 26K protein has been demonstrated to possess a general cytolytic activity (Thomas, W.E. and Ellar, D.J. [1983] FEBS Letters 154:362-368) and has been reported to be the specific dipteran $\delta$-endotoxin (Yamamato, T., Iizuka, T. and Aaronson, J.N. [1983] Current Microbiology 9:297-284; Armstrong, J.L., Rohrmann, G.F., and Beaudreau, G.S. [1985] J. Bacteriol. 161:39-46). The gene encoding the 26K protein was recently cloned and the recombinant gene product was reported to be cytolytic in a cell culture assay and toxic to mosquito larvae (Ward, E.S., Ellar, D.J., and Todd, J.A. [1984] FEBS Letters 175:377-382).

Disclosed and claimed are novel polypeptides with a biological property of specific toxicity to larvae of the Dipteran family. These novel polypeptides can be unambiguously identified in alkaline extracts of Bti parasporal crystals by the following properties:

35K Polypeptide:

1. a molecular weight of about 35000d±3000d as determined by gel electrophoresis in the presence of the denaturing detergent, sodium dodecyl sulfate;
2. an isoelectric point of about 6.5±0.3 as determined by isoelectric focusing in a continuous linear pH gradient;
3. the ability to bind to a variety of erythrocyte membranes without effecting gross hemolysis (lysing of the cells) or losing toxicity; and
4. toxicity to Dipteran larvae with an $LC_{50}$ of less than 0.5 $\mu$g/ml.

66K Polypeptide:

1. a molecular weight of about 66000d±4000d as measured by SDS-PAGE;
2. which upon proteolytic digestion gives rise to a polypeptide having the following characteristics:
   (a) a molecular weight of about 35000d±3000d as determined by gel electrophoresis in the presence of the denaturing detergent, sodium dodecyl sulfate;
   (b) an isoelectric point of about 6.5±0.3 as determined by isoelectric focusing in a continuous linear pH gradient;
   (c) the ability to bind to a variety of erythrocyte membranes without effecting gross hemolysis (lysing of the cells) or losing toxicity; and

(d) toxicity to Dipteran larvae with an $LC_{50}$ of less than 0.05 $\mu$g/ml;

and

3. has specific toxicity to Dipteran larvae.

The novel polypeptides of the subject invention can be isolated from Bti crystal extracts. The details of the isolation and characterization procedures used in the subject invention are presented in the Examples, infra.

The Bti crystals can be obtained from any Bti organism. For example, a suitable source is Bacillus thuringiensis var. israelensis, ATCC 35646, available from the American Type Culture Collection, located in Rockville, MD, USA.

The novel polypeptides of the subject invention can be formulated in a variety of ways for treating the situs of Dipteran larvae.

The property of membrane binding as described herein makes possible the incorporation of these polypeptides, and other B.t.i. polypeptides active against Dipteran larvae, into a particulate form by absorption to phospholipid vesicles. This can provide an effective means of delivery of the larvacidal polypeptide(s) to the filter feeding dipteran larvae. Specific absorption can occur by passive binding of the larvacidal polypeptide(s) to natural or synthetic lipid vesicles in an aqueous suspension. Lipid vesicles may be generated from natural sources such as erythrocyte or other cellular membranes or from synthetic compounds such as lecithin or other lipid compounds capable of forming colloidal suspensions in water. Particulate absorbents could also include latex beads (Schell, D., Pfannenstiel, M.A., and Nickerson, K.W. (1984) Science 223:1191-1193) or low density polyethylene vesicles (WPI Acc. No. 85-101445/17).

The polypeptides can be formulated as wettable powders, granules or dusts, by mixing with various inert materials, such as inorganic minerals (phyllosilicates, carbonates, sulfates, phosphates, and the like) or botanical materials (powdered corncobs, rice hulls, walnut shells, and the like). The formulations may include spreader-sticker adjuvants, stabilizing agents, other pesticidal additives, or surfactants. Liquid formulations can be aqueous-based or non-aqueous and employed as foams, gels, suspensions, emulsifiable concentrates, or the like. The ingredients may include rheological agents, surfactants, emulsifiers, dispersants, or polymers.

The pesticidal concentration will vary widely depending upon the nature of the particular formulation, particularly whether it is a concentrate or to be used directly. The pesticide may be present at less than 1% by weight and may be 100% by weight. The dry formulations will have from about 1-95% by weight of the pesticide while the liquid formulations will generally be from about 1-60% by weight of the solids in the liquid phase.

The formulations can be applied to the environment of the Dipteran larvae, e.g., plants, soil or water; by spraying, dusting, sprinkling or the like.

Following are examples which illustrate procedures, including the best mode, for practicing the invention. These examples should not be construed as limiting. All percentages are by weight and all solvent mixture proportions are by volume unless otherwise noted.

Example 1--Separation of mosquito larvae activity from hemolytic activity

Parasporal crystals from Bti are purified by means of centrifugation on a density gradient formed with Renografin® (Tyrell, D.J., Davidson, L.I., Bulla, L.A., and Ramoska, W.A. [1979] Applied and Environmental Microbiology 38:656-658). This technique readily separates the crystals from the spores as well as from other cellular debris. The purified crystals are extracted with a buffer comprising 50 mM $Na_2CO_3$, pH 10.5 for 1 hr at 37°C. This extraction procedure results in the release of a variety of soluble proteins including species with molecular weights of ~65000d and 26000d as well as a complex of several proteins in the molecular weight range of 32000d-36000d. These proteins can be visualized and molecular weight determinations made by polyacrylamide gel electrophoresis in the presence of sodium dodecyl sulfate (SDS-PAGE) followed by staining with Coomassie blue R250, as is well known in the art (Laemmli, U.K., [1970] Nature 227:680-685). These soluble extracts have been demonstrated to have at least two biological activities. One is a general cytolytic activity which is time and temperature dependent and is capable of lysing a wide variety of cells in culture. The other is a highly specific toxic activity toward mosquito larvae. The general cytolytic activity can be assayed most conveniently by hemolysis of mammalian erythrocytes (RBCs). The assay can be conducted as follows:

1. Fresh or stored mammalian (human, rabbit, or rat work equally well) red blood cells are washed in an isotonic (25 mM phosphate, pH 6.8; 125 mM NaCl) saline solution (PBS) to remove plasma. The cells are collected by centrifugation (1000g for 10 min) and 1 ml of the pelleted RBCs is suspended in 10 ml of PBS to make a suspension that is defined as 10%.

2. The samples to be tested are placed in an isotonic buffer. The choice of the buffer itself is not critical but it is essential that the solution be isotonic to avoid lysis of the cells by osmotic shock.

3. 100 $\mu$l of the 10% suspension of RBCs and 100 $\mu$l of the sample to be tested are combined.

If a large number of assays are to be conducted (when assaying column fractions for instance) it is convenient to carry this out in a 96 well microtiter plate. The assay samples are incubated with shaking for 30 min at 37°C. Following this the cells are allowed to settle and the assay is scored either qualitatively ( + + = total lysis, + = partial lysis, or 0 = no lysis) or quantitatively by diluting 25 $\mu$l of the assay supernate to 1 ml with water and determining the optical density at 410 nm.

The larvacidal activity can be assayed by adding an aliquot of the sample to be tested directly to a vial containing 5 mosquito larvae in 5 ml of deionized water. This bioassay is read after 24 hr and the $LC_{50}$ is determined from a dilution series of an individual sample.

Separation of the general cytolytic activity from the Dipteran toxicity is accomplished by gel filtration on a column of Ultrogel® AcA54 (LKB, Bromma, Sweden), or equivalent, in a buffer comprising 50 mM Tris-HCl, pH 8.0, 150 mM NaCl. The column dimensions are 2 x 75 cm and elution is done at a rate of ~7 ml/hr. 3.7 ml fractions are collected and the elution is monitored at 280 nm.

A number of peaks of 280 nm absorbance are noted, but the Dipteran toxic activity is resolved into two peaks centered at Fractions 21 (Peak I) and 30 (Peak II), while the hemolytic activity is confined to a single peak centered at Fraction 36. No toxicity to mosquito larvae is detectable in Fraction 36 at protein concentrations as high as 10-15 $\mu$g/ml; conversely, little or no hemolytic acitivity is associated with the toxic peaks.

SDS-PAGE analysis of these toxic peak fractions reveals the presence of two principal proteins in Peak I. The molecular weights of these bands are determined to be 66000d±4000d and 35000d±3000d by comparison with standards. Upon standing, the 66000d band is observed to disappear while the 35000d band increases in intensity. No significant loss of toxicity is detectable. This suggests that the 66000d protein is a precursor to a smaller protein of MW 35000 and that the smaller fragment retains all of the toxicity of the larger protoxin.

SDS gel analysis of Peak II reveals no detectable trace of the 66000d protein; instead a complex of three or more proteins is seen with molecular weights ranging from 32000d to around 36000d. No proteins of lower molecular weight are detectable.

The peak of hemolytic activity centered around Fraction 36 is seen to contain a 26000d protein as the major constituent. This band reacts strongly with antisera raised against the purified 26K protein which is the major species present in Bti crystals. An important observation is that no significant cross-activity is noted between these 26K specific antisera and any of the proteins present in Peaks I or II. This indicates that the 26000d (26K) protein which is the principal component of the hemolytic peak is unrelated to the protein(s) responsible for Dipteran toxicity.

Example 2--Binding of Dipteran toxic activity to lipid membranes

Erythrocytes from which all the cytoplasmic protein had been removed (RBC ghosts) were used to assess the ability of Bti soluble proteins to bind to cell membranes. These RBC ghosts were prepared by osmotic shock which consisted of repeated cycles of exposure to hypotonic (10 mM phosphate, pH 7.2) followed by hypertonic (110 mM phosphate, pH 7.2) buffer following the method of Dodge et al. (Dodge, J.T., Mitchell, C., and Hanahan, D.J. [1963] Arch. Biochem. Biophys. 100:119-127). Lysis was judged complete when the RBC ghosts were colorless, indicating complete removal of the hemoglobin and other cytoplasmic components. The ghost membranes were collected by centrifugation (10,000g for 30 min) and used in protein absorption (binding) experiments with both unfractionated crystal extracts and partially purified column fractions.

An unfractionated crystal extract made as described in Example 1 (100 $\mu$l) was incubated with 25 $\mu$l of ghost membranes for 30 min at 37°C. The absorbed and preabsorbed supernates were then tested for hemolytic activity and larvacidal activity. It was found that absorption with RBC ghost membranes resulted in the loss of both the hemolytic and the toxic activity from the extracts, suggesting that the toxic and hemolytic factors had become bound to the membranes. This was confirmed in the case of the toxic activity by the demonstration that, following absorption, the ghost membranes became highly toxic to moxquito larvae, i.e., the toxic activity which was depleted from the extract was concentrated in the ghost membranes. Binding to the cell membranes has the effect of increasing the specific activity of the toxin probably because the filter feeding Dipteran larvae can more efficiently ingest it in a particulate form. Visual examination of the Coomassie stained SDS polyacrylamide gel of the soluble extract following absorption revealed the apparent selective removal of the 26K protein. The 26K protein band was found to be associated with the RBC ghost membrane fraction.

In order to identify any other species which might be selectively associating with the RBC ghosts, the proteins present in the unfractionated extract were labeled with $^{125}$I to serve as a radioactive tracer. Incubation of these radioactive extracts with the RBC ghosts followed by SDS-Page and autoradiography revealed that two proteins were specifically and tightly bound to the ghost membrane. The molecular weights of these bound proteins were estimated by SDS-PAGE to be 26000d and 35000d. The 26000d protein is undoubtedly the protein of the same molecular weight (26K) which was observed by SDS-PAGE to be removed from the extract and bound to the membrane fraction. The 35000d species is most likely the small protein detectable in toxic Peak I and is also present in toxic Peak II but is masked by unrelated proteins of similar molecular weight. Loss of the 35000d protein from the unfractionated extract would not have been apparent because there are a number of Bti crystal proteins present in this molecular weight range.

The 26000d protein and the 35000d protein were compared by the technique of peptide mapping as described by Cleveland et al. (Cleveland, D.W., Fischer, S.G., Kirschner, M.W., and Laemmli, U.K. [1977] J. Biol. Chem. 252:1102-1106) and no homology was demonstrable between the two proteins. This is consistent with the finding that the antisera directed toward the 26K protein did not cross-react with the 35000d protein or any proteins present in Peak II. It is concluded that the tight binding of both of these proteins to RBC ghost membranes is coincidental and not related to homology between the molecules.

On the basis of these data it seemed probable that the 35000d protein (35K) was a unique Dipteran-specific toxin. If this was true it should be possible to specifically absorb this protein from Peak II using RBC ghosts. Fraction 30 from the gel filtration experiment described above was radioactively labeled with $^{125}$I and absorbed with RBC ghosts. SDS-PAGE followed by autoradiographic analysis revealed the presence of only one radioactive band binding to the ghost membranes. The molecular weight of this protein was determined to be 35000d. Similar experiments done with unlabeled extracts revealed that the toxic activity was specifically removed from Peak II by absorption with RBC ghosts and became concentrated in the lipid membrane following absorption. It is concluded that this 35000d protein is the 35K protein identified above.

Example 3--Determination of isoelectric point

RBC ghost membranes to which purified, radioactively labeled 35K protein had been bound were analyzed by two-dimensional electrophoresis as described by O'Farrell (O'Farrell, P.H. [1975] J. Biol. Chem. 250:4007-4021) in which separation in the first dimension is accomplished by isoelectric focusing and separation in the second dimension by molecular weight. Location of a protein in the pH gradient gives an accurate determination of isoelectric point (pI). Autoradiography of the two-dimensional gel confirmed the presence of a single protein which had an isoelectric point of 6.5 The other proteins in the extract with molecular weights near 35000d also had similar isoelectric points but displayed no binding whatsover to the ghost membranes.

The above Examples unambiguously identify a novel Dipteran toxin with unique properties. These properties include a moleculer weight of about 35000±3000d, an isoelectric point of about 6.5±0.3, and the ability to bind to lipid membranes. In addition to the 35K protein there may be at least one additional protein with specific toxicity to Dipteran larvae. This species, with a molecular weight of about 66000±4000d, appears to be a precursor to the 35000d±3000d protein and may require processing for activity. In any case, no loss of activity was noted upon conversion of the 66000d protein to the 35000d protein, demonstrating that the 35000d fragment contains the entire domain of Dipteran toxicity. No smaller fragments with larvacidal activity were detectable and no evidence was found for homology between the 26000d hemolytic protein and either the 35000d Dipteran toxic or the 66000d precursor.

**Claims**

1. Bacillus thuringiensis israelensis protein characterized by being substantially free of non-selective Bacillus thuringiensis israelensis cytolytic factors and having:

    (a) a molecular weight of about 35000d±3000d as determined by gel electrophoresis in the presence of the denaturing detergent, sodium dodecyl sulfate;

    (b) an isoelectric point of about 6.5±0.3 as determined by isoelectric focusing in a continuous linear pH gradient;

    (c) the ability to bind to a variety of erythrocyte membranes without effecting gross hemolysis or losing toxicity; and

    (d) toxicity to Dipteran larvae with an $LC_{50}$ of less than 0.05 $\mu$g/ml.

2. A Dipteran larvacidal composition comprising a larvacidally effective amount of the protein of claim 1 in a mixture with an inert carrier.

3. A Dipteran larvacidal composition according to claim 2 wherein the protein is absorbed to natural or synthetic phospholipid vesicles.

4. A method for killing larvae of the Dipteran family which comprises treating said larvae, or the situs of said larvae, with an effective anti-larvae amount of the protein of claim 1.

5. A method for killing larvae of the Dipteran family which comprises treating said larvae, or the situs of said larvae, with an effective anti-larvae amount of the composition of claims 2 to 3.

**Patentansprüche**

1. Bacillus thuringiensis israelensis Protein, dadurch gekennzeichnet, daß es im wesentlichen frei ist von nicht-selektiven zytolytischen Faktoren von Bacillus thuringiensis israelensis und daß es:

(a) ein Molekulargewicht von etwa 35000d ± 3000d hat, bestimmt mit Gelelektrophorese in Gegenwart des denaturierenden Detergenz Natriumdodecylsulfat;

(b) einen isoelektrischen Punkt von etwa 6,5 ± 0,3 hat, bestimmt mit Isoelektrofokussierung mit einem kontinuierlichen linearen pH-Gradienten;

(c) die Fähigkeit hat, an eine Vielzahl von Erythrozytenmembranen zu binden, ohne eine starke Hämolyse zu bewirken oder die Toxizität zu verlieren; und

(d) die Toxizität gegenüber Zweiflüglerlarven hat mit einer $LC_{50}$ von weniger als 0,05 $\mu$g/ml.

2. Zweiflüglerlarvizidzusammensetzung, umfassend eine larvizid wirksame Menge des Proteins von Anspruch 1 in Mischung mit einem inerten Träger.

3. Zweiflüglerlarvizidzusammensetzung gemäß Anspruch 2, worin das Protein an natürliche oder synthetische Phospholipidvesikel absorbiert ist.

4. Verfahren zum Töten von Larven der Zweiflüglerfamilie, umfassend, daß man die Larven oder den Aufenthaltsort der Larven mit einer wirksamen, gegen die Larven wirkenden Menge des Proteins von Anspruch 1 behandelt.

5. Verfahren zum Töten von Larven der Zweiflüglerfamilie, umfassend, daß man die Larven oder den Aufenthaltsort der Larven mit einer wirksamen, gegen die Larven wirkenden Menge der Zusammensetzung der Ansprüche 2 bis 3 behandelt.

**Revendications**

1. Protéine de Bacillus thuringiensis israelensis caractérisée comme étant substantiellement exempte de facteurs cytolytiques non sélectifs de Bacillus thuringiensis israelensis et ayant

a) un poids moléculaire d'environ 35 000 d ± 3000 d comme déterminé par électrophorèse sur gel en présence du détergent dénaturant le dodécylsulfate de sodium ;

b) un point isoélectrique d'environ 6,5 ± 0,3 comme déterminé par focalisation isoélectrique dans un gradient de pH continu et linéaire ;

c) l'aptitude de se lier sur de nombreuses membranes d'érythrocytes sans induire d'hémolyse importante ou sans perdre sa toxicité; et

d) une toxicité pour les larves de diptères avec un $CL_{50}$ inférieure à 0,05 $\mu$g / ml.

2. Une composition larvicide contre les diptères comprenant une quantité efficace comme larvicide de la protéine de la revendication 1 en mélange avec un support inerte.

3. Une composition larvicide contre les diptères selon la revendication 2, dans laquelle la protéine est absorbée dans des vésicules de phospholipides naturels ou synthétiques.

4. Procédé pour détruire des larves de diptères qui comprend le traitement desdites larves, ou l'habitat desdites larves, par une quantité antilarvaire efficace de la protéine de la revendication 1.

5. Procédé pour détruire des larves de diptères qui comprend le traitement desdites larves, ou l'habitat desdites larves, par une quantité antilarvaire efficace de la composition des revendications 2 à 3.